# EUROPEAN PATENT APPLICATION

(11) **EP 0 968 693 A2**
(43) Date of publication of application: **05.01.2000**
(21) Application number: 99305066.5
(22) Date of filing: 28.06.1999
(51) Int. Cl.: A61F 2/46

(54) **Inserter device**

(30) Priority: 29.06.1998 GB 9814026
(71) Applicant: Johnson & Johnson Medical Limited, Ascot, Berkshire SK5 9EY (GB)
(72) Inventor: Stungo, Benedict Guy, Leeds LS1 3LA (GB)
(74) Representative: Fisher, Adrian John

(57) **Abstract**

An inserter device (1) is described with a handle (4) and a shaft (10). A lever (2) is pivotally attached to the shaft (10) at its distal end (34). The lever (12) has an operating thumb plate (20) adjacent the handle (4) of the inserter device (1). Operation of the lever (12) to press the thumb plate (20) towards the handle (4) results in a mechanical interlock at the distal end (32) of the shaft (10) by means of a pivoting of a projection (40) at the end of the lever (12) out of alignment with corresponding projections (38) at the end of the shaft (10).

## Description

This invention relates to an inserter device for inserting a prosthetic component in a bone cavity. Prosthetic components such as femoral stem components are inserted into a bone cavity in a cement mantle. It is important that an inserter device can be detached from the implanted prosthetic component without transferring loads to the component. This is particularly relevant as surgeons are concerned that the act of detaching and removing an inserter device could misplace the prosthetic component within the cement mantle, creating routes for debris ingress between the mantle and the prosthetic component.

The inserter device of the present application has an aim of enabling a prosthetic component, for example a femoral stem component, to be rigidly held whilst it is implanted into a cement mantle. The inserter device of the present application has the further aim of allowing the prosthetic component to be held in a single handed fashion.

It is advantageous for rotational loads to be transferred from a handle of an inserter device to a prosthetic component, to control version of the prosthetic component. It is further advantageous to enable the prosthetic component to be loaded along its centre line, thus ensuring that the implantation loads do not rotate the prosthetic component. Furthermore, it is advantageous to ensure that no loads are transferred to the prosthetic component whilst an inserter device is being removed, thus ensuring that the prosthetic component is left in the desired position within the cement mantle.

According to the present invention there is provided an inserter device for inserting a prosthetic component in a bone cavity comprising a shaft having a distal end and a proximal end, a handle at the proximal end of the shaft, and a mechanical interlock at the distal end of the shaft, wherein the mechanical interlock is operated by an operating means disposed at the handle.

Preferably, the mechanical interlock is in the form of at least two relatively moveable members moveable between an aligned position providing an unlocked position and an unaligned position providing a locked position. Preferably, the members are relatively pivotable.

The mechanical interlock may be in the form of members comprising two projections at the distal end of the shaft and a single projection at a distal end of the operating means. Preferably, the single projection at the distal end of the operating means is disposed between the two projections at the distal end of the shaft.

Preferably, the projections have a rectangular cross-section and, in their aligned position form a continuously rectangular ridge.

Preferably, the operating means is in the form of a lever. Preferably, the lever is pivoted in relation to the shaft with a pivot point adjacent the distal end of the shaft. The lever may extend from the distal end of the shaft to the handle and may be operated at the handle. Preferably, a plate is provided at the handle end of the lever.

Preferably, the handle is profiled to correspond to a single hand grip. Preferably, the operating plate of the operating means is adapted for comfortable operation by an operator's thumb.

An embodiment of an inserter device in accordance with the present invention is now described, by means of example only, with reference to the drawings in which:

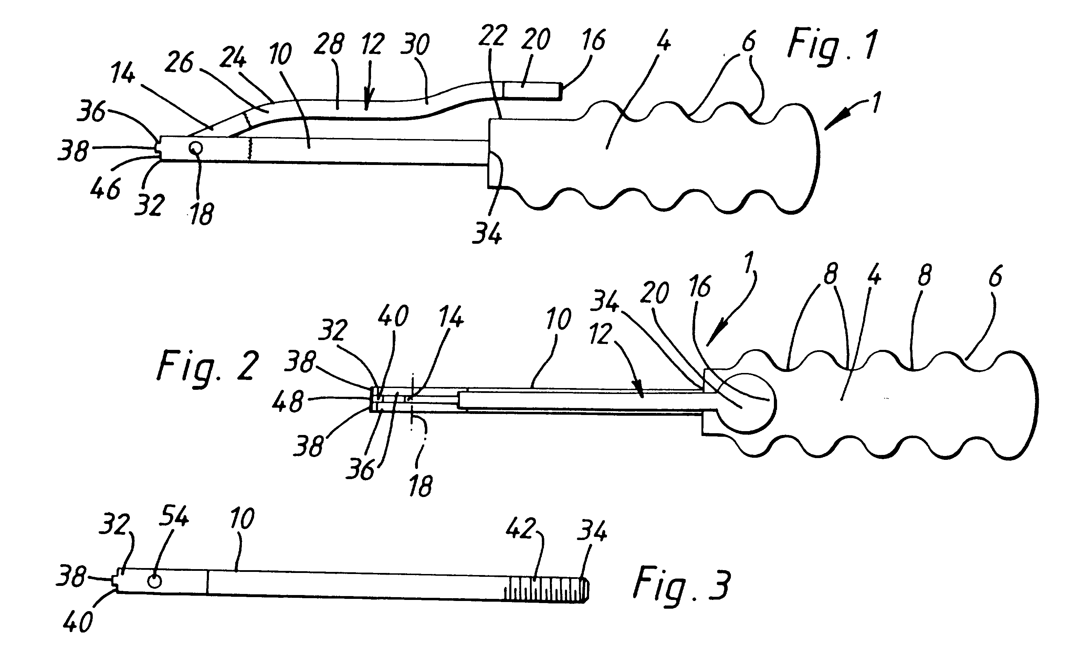

Figure 1 is a side elevation of an inserter device in accordance with the present invention;
Figure 2 is a plan view of the inserter device of Figure 1;
Figure 3 is a side elevation of the shaft of the inserter device of Figure 1;

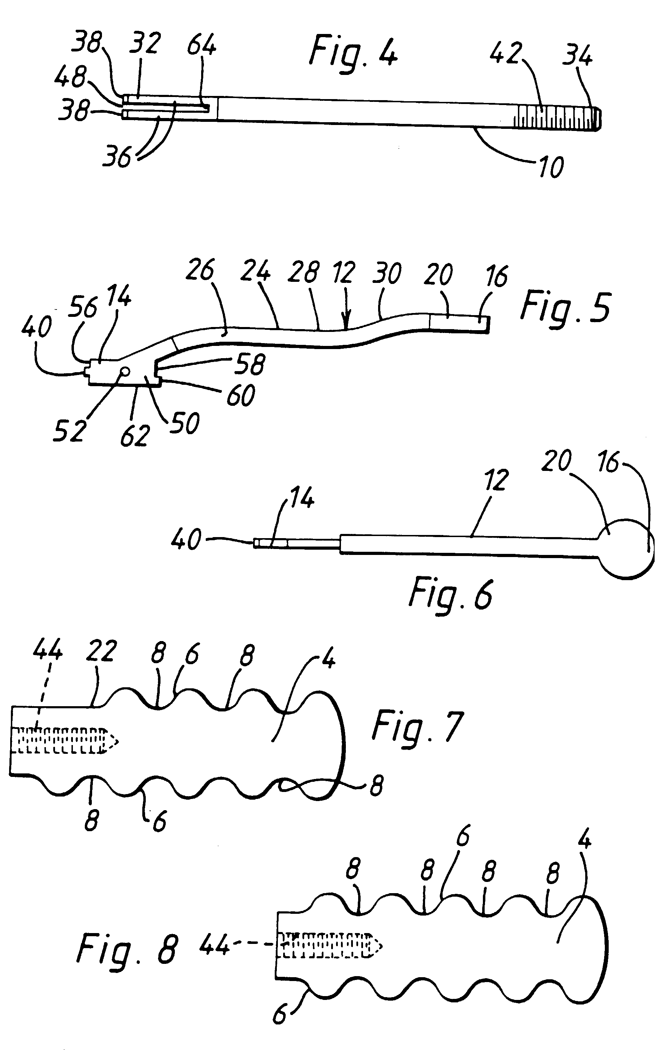

Figure 4 is a plan view of the shaft of Figure 3;
Figure 5 is a side elevation of a lever of the inserter device of Figure 1;
Figure 6 is a plan of the lever of Figure 5;
Figure 7 is a partial vertical cross-section of a handle of the inserter device of Figure 1; and
Figure 8 is a partial horizontal cross-section of the handle of Figure 7.

Referring to the drawings, an inserter device 1 has a handle 4 and a shaft 10. The handle 4 is designed to be held by a single hand of an operator and is profiled 6 for a single-handed grip by the provision of recesses 8 for the fingers of the operator's hand.

The shaft 10 has a proximal end 34 and a distal end 32. The proximal end 34 is attached to the handle 4 by means of a screw threaded connection. The proximal end 34 of the shaft 10 has a screw thread 42 which corresponds to a screw threaded recess 44 in the handle 4.

The distal end 32 of the shaft 10 is in the form of a two pronged fork 36 which extends a short distance from the distal end 32 back along the shaft 10.

The end faces 46 of the prongs of the fork 36 are flat with rectangular projections 38 extending therefrom. The rectangular projections 38 on each of the prongs of the fork 36 are aligned such that their top, end and bottom surfaces are in the same planes. The rectangular projections 38 form a rectangular ridge with a gap 48 in the middle of the ridge.

The inserter device 1 also has a lever 12 with a distal end 14 and a proximal end 16. The lever 12 has a curved profile 24.

The lever 12 is pivotally attached to the shaft 10 by pivot pin 18 with a pivotal axis which extends transversely mid way along the length of the fork 36 at the distal end 32 of the shaft 10. The lever 12 and the shaft 10 are pivoted about points a short distance from their distal ends 14, 32, through pivot holes 52, 54.

The lever 12 has a foot portion 50 of generally rectangular shape in side elevation which is insertable between the two prongs of the fork 36 of the distal end 32 of the shaft 10. The foot portion 50 of the lever 12 has a central pivot hole 52 through which the pivot pin 18 is inserted. The foot portion 50 has an end face 56 with a rectangular projection 40 extending therefrom. The rectangular projection 40 corresponds in shape and size to the rectangular projections 38 on the end faces 46 of the two prongs of the fork 36 of the shaft 10. The foot portion 50 has a rear opposing face 58 opposite the end face 56. The rear face 58 has a projection 60 extending from the bottom edge 62 of the foot portion 50. The rear face 58, in use, lies adjacent the closed end 64 of the fork 36 of the shaft 10 with the projection 60 extending further than the closed end 64 to provide a stop for the lever 12 preventing movement of the lever 12 away from the shaft 10.

In a first position, the lever 12 extends generally parallel to the shaft 10. A first curved portion 26 of the lever 12 curves from the pivot hole 52 of the foot portion 50 at an angle to the longitudinal not axis of the shaft 10. A second portion 28 of the lever 12 extends, in the first position, generally parallel to the shaft 10. A further curved portion 30 curves the lever 12 in an 'S' shape away from the shaft 10 and ends in a plate 20 at the proximal end of the lever 12. The plate 20 is round for comfortable accommodation of a thumb of the operator and terminates adjacent the handle 4. The handle 4 has a non-profiled portion 22 adjacent the plate 20. In this first position, the plate 20 is a distance from the un-profiled portion 22 of the handle 4.

In the first position, the rectangular projection 40 at the distal end 14 of the lever 12 is aligned with the rectangular projections 38 of the fork 36 of the shaft 10. The projection 60 from the rear face 58 of the foot portion 50 of the lever 12 abuts the shaft 10 preventing the lever 12 from being pivoted further away from the shaft 10.

A second position is provided in which the lever 12 is pivoted towards the shaft 10 such that the plate 20 at the proximal end 16 of the lever 12 touches the un-profiled portion 22 of the handle 4. In this second position, the rectangular projection 40 on the end face 56 of the foot portion 50 of the lever 12 is pivoted out of alignment with the rectangular projections 38 of the prongs of the fork 36 of the shaft 10. In this second position, the projection 60 has moved out of abutment with the shaft 10.

The inserter device 1 is operated by a surgeon who holds the handle 4 in a single-handed grip with his fingers in the recesses 8 of the handle 4 and his thumb on the plate 20 of the lever 12. With the inserter device 1 in its first position, the rectangular projection 40 of the lever 12 and the rectangular projections 38 of the shaft 10 are aligned to form a rectangular ridge which is insertable in a groove (not shown) in a prosthetic component to be implanted. The elongate ridge formed by the projections 38, 40 enables rotational loads to be transferred from the handle 4 to the prosthetic component to control the position of the prosthetic component. In addition, the prosthetic component can be loaded along its centre line ensuring that the implantation loads do not rotate the prosthetic component.

Once the ridge formed by projections 38, 40 is in the correct position in the groove of the prosthetic component, the surgeon can apply pressure to the plate 20 via his thumb. If the plate 20 is compressed against the un-profile portion 22 of the handle 4, the rectangular projection 40 of the lever 12, pivots out of alignment with the rectangular projections 38 of the shaft 10 and pressure is inserted on the sides of the groove of the prosthetic component holding the inserter device 1 in a mechanical interlock with the prosthetic component. The mechanical interlock is sufficient to hold the prosthetic component in a suitably secure fashion such that the loads required to implant the prosthetic component can be transferred from the inserter device 1 to the prosthetic component. Once the prosthetic component is in place, the removal of the thumb pressure by the surgeon removes the mechanical interlock and thus the inserter device 1 can be removed from the groove of the prosthetic component without disturbing the prosthetic component. The removal of the mechanical interlock ensures that no loads are transferred to the prosthetic component whilst the inserter device 1 is being removed, thus ensuring that the prosthetic component is left in the desired position within the cement mantle in the bone cavity of the patient.

Modifications and improvements can be made to the foregoing without departing from the scope of the present invention.

## Claims

1. An inserter device for inserting a prosthetic component in a bone cavity comprising a shaft having a distal end and a proximal end, a handle at the proximal end of the shaft, and a mechanical interlock at the distal end of the shaft, wherein the mechanical interlock is operated by an operating means disposed at the handle.

2. An inserter device as claimed in claim 1, wherein the mechanical interlock is in the form of at least two relatively moveable members moveable between an aligned position providing an unlocked position and an unaligned position providing a locked position.

3. An inserter device as claimed in claim 2, wherein the members are relatively pivotable.

4. An inserter device as claimed in any of claims 1 to 3, wherein the mechanical interlock is in the form of members comprising two projections at the distal end of the shaft and a single projection at a distal end of the operating means.

5. An inserter device are claimed in claim 4, wherein the single projection at the distal end of the operating means is disposed between the two projections at the distal end of the shaft.

6. An inserter device as claimed in claim 4 or claim 5, wherein the projections have a rectangular cross-section and, in their aligned position form a continuously rectangular ridge.

7. An inserter device as claimed in any of the preceding claims, wherein, the operating means is in the form of a lever.

8. An inserter device as claimed in claim 7, wherein the lever is pivoted in relation to the shaft with a pivot point adjacent the distal end of the shaft.

9. An inserter device as claimed in claim 7 or claim 8, wherein the lever extends from the distal end of the shaft to the handle and is operated at the handle.

10. An inserter device as claimed in any of claims 7 to 9, wherein a plate is provided at the handle end of the lever.

11. An inserter device as claimed in any of the preceding claims, wherein the handle is profiled to correspond to a single hand grip.

12. An inserter device as claimed in claim 10, wherein the operating plate of the operating means is adapted for comfortable operation by an operator's thumb.
